# EUROPEAN PATENT APPLICATION

(11) **EP 1 475 078 A1**
(43) Date of publication of application: **10.11.2004**
(21) Application number: 04252684.8
(22) Date of filing: 07.05.2004
(51) Int. Cl.: A61K 7/46

(54) **Polymer particles and methods for their preparation and use**

(30) Priority: 09.05.2003 US 434981
(71) Applicant: INTERNATIONAL FLAVORS & FRAGRANCES INC., New York New York 10019 (US)
(72) Inventor: Popplewell, Lewis Michael, Monmouth County New Jersey 07751 (US); Bryant, Cory Michael, New York County New York 10011 (US)
(74) Representative: Mercer, Christopher Paul

(57) **Abstract**

A particle composition comprising a water-soluble carrier such as starch, a structuring agent such as an ester terminated polyamide and fragrance or flavor is disclosed. Methods of making and using the particles are also disclosed. The particles are desirable for providing fragrance to various products such as powdered detergents.

## Description

### FIELD OF INVENTION

The invention relates to particles containing starches or other water soluble carriers, ester terminated polyamides or other hydrophobic structuring materials and fragrances or flavors, as well as methods for the preparation and use of such particles.

### BACKGROUND OF THE ART

In recent years, the food, cosmetics, and fragrance industries have found interest in encapsulated products. Such products allow incorporation of fragrance in small distinct packages that can be dispersed throughout a dispersion medium. Such encapsulated products are generally formed from carriers or encapsulants that are not miscible or compatible with the active agent. For example, hydrophilic substances are often used to surround and encapsulate hydrophobic substances. This type of particle is a core-shell type particle, or in some cases a multi-core type particle. As suggested by the name, two discrete phases make up the particle. The core and shell are each maintained as individual components rather than a continuous phase.

Most encapsulated flavors or fragrances are formed via spray drying. Spray drying typically involves the use of water-soluble polymeric encapsulants such as gum arabic, modified starch, and/or dextrin as the main carrier materials. These materials are often used in combination with varying amounts of plasticizers, gellants, and surface-active polymers to control processing, and more importantly, product characteristics. As discussed above, the flavor or fragrance to be encapsulated is normally not miscible with the water-soluble carrier system selected, although some flavor and fragrance materials can plasticize the carriers appreciably. Many variations of spray-dried carriers are used by those familiar with the art to control product stability and release. These variations focus almost exclusively on the water soluble carrier and its properties in the dry state, or on control of the kinetic process of hydration/dissolution, as opposed to attempting to control the release of flavor or fragrance once the carrier is dissolved.

A continuing challenge in the area of flavor and fragrance delivery, then, is the identification of encapsulation systems that provide release characteristics suitable for a specific application and environment.

### SUMMARY OF THE INVENTION

A particle composition comprising discrete particles containing fragrances or flavors, ester terminated polyamide (ETPA), or other similarly functional structuring materials, and water-soluble carriers is described herein.

In a preferred embodiment, the particles comprise from about 20 to about 90 weight percent carrier, structuring material from about 5 to about 40 weight percent and fragrance or flavor from about 5 to about 40 weight percent on a dry basis. The particles are preferably formed by creating a mixture of water soluble carrier in a solvent, preferably water, preferably at an elevated temperature. A second mixture is formed by mixing the structuring material and flavor or fragrance, preferably with heating. The two mixtures are then admixed, preferably under high shear mixing or homogenization to form a dispersion and the particles are then obtained by drying.

### BRIEF DESCRIPTION OF THE DRAWINGS

Figure 1 is a depiction of a single particle of the carrier material with the fragrance and ETPA distributed throughout the particle.
Figure 2 is a graphical presentation of the average intensity of the headspace odor emitted above an open wash basin containing cloth that is soaking in detergent containing added fragrance particles.

### DETAILED DESCRIPTION

The invention essentially consists of the surprising discovery that specific hydrophobic, meltable polymers/materials can be used to create discrete polymer-flavor/fragrance beads within a dried water-soluble carrier. The discrete polymer particles are distributed throughout a continuous phase of water-soluble carrier. Particles with this structure provide two main, useful, benefits. First, the water-soluble carrier can be designed to provide appropriate protection to the volatile, or otherwise sensitive flavor/fragrance materials. Second, the structured beads, once released from the carrier via its dissolution, control diffusion of the flavor/fragrance into the surrounding environment. In a preferred embodiment, spray drying is used to form the particles since the steps of emulsion formation and drying are fundamental characteristics of the process. Another preferred embodiment provides the use of ETPA as the structuring agent, as it has a high affinity for fragrances, and can effectively control their evaporation/diffusion. A further advantage of the use of ETPA is that it is a meltable polymer allowing further protection of a fragrance material when employed in heated environments found in situations such as laundering of clothing.

The water-soluble carrier used in this invention includes starches, dextrins, hydrocolloids, low molecular weight plasticizers, and surface active agents. Starch, as used in this application, includes starch, chemically modified starch, maltodextrin and the like. Suitable hydrocolloid materials include gum arabic, cellulosic polymers, gelatin and alginates; suitable low molecular weight plasticizers include polyols such as glycerine, sugars such as sucrose and dextrin; and suitable surface active agents include pluronic polyols, polysorbates, lecithins, ethoxylates, laurates, oleates, stearates, and lactylates. As those with skill in the art recognize, a mixture of carrier materials appropriate for the particular application can be employed in the present invention.

The dried particles of the present invention contain from about 20 to about 90 weight percent water soluble carrier, preferably starch, preferably from about 30 to about 80 and most preferably from about 40 to about 70 weight percent starch or carrier materials.

The preferred structuring agents used in the present invention include waxes, fats and ETPA. The common characteristics of these materials are that they are normally crystalline, when above their melting point are reasonably soluble in the flavor or fragrance, and upon cooling substantially re-crystallize in the presence of the flavor or fragrance. The re-crystallization creates the structured flavor/fragrance bead embedded within the soluble carrier. Of course, other structuring materials which create a bead via a substantial increase in viscosity without re-crystallization may also be used, but are less preferred.

Ester terminated polyamides (ETPA) are disclosed in U.S. Patent Nos. 5,783,657; 5,998,570; 6,111,055; 6,214,063 and 6,268,466. Similarly, amide terminated polyamides (ATPA) are disclosed in U.S. Patent No. 6,268,466. Both ETPA and ATPA are commercially available materials from Arizona Chemical, of Jacksonville, Florida. As used throughout the attached specification and claims ETPA is meant to include ETPA, ATPA and mixtures of ETPA and ATPA.

Other meltable structuring materials that may be used in the present invention to create the internal flavor/fragrance bead phase include triglycerides that melt above approximately 25°C, waxes such as beeswax, carnauba wax, paraffin waxes; stearyl alcohol, and low molecular weight polyethylene. Highly desirable properties of these materials are that they melt or soften sufficiently at temperatures below about 120°C in order to promote gentle combination with flavor/fragrance materials, that they form a largely continuous phase with flavor/fragrance materials once melted or softened, and that they form a reasonably solid structure with substantial amounts of entrapped fragrance/flavor once cooled to ambient temperature. These properties jointly allow for the efficient incorporation of flavor/fragrance, as well as the desired structural and controlled release characteristics.

The level of structuring agent, such as ETPA, provided in the dried particles of the invention is from about 10 to about 40 weight percent of the particle, preferably from about 15 to about 35 and most preferably from about 20 to about 30 weight percent of the particle.

Many types of fragrances can be employed in the present invention, the only limitation being the compatibility with the other components being employed. Suitable fragrances include but are not limited to fruits such as almond, apple, cherry, grape, pear, pineapple, orange, strawberry, raspberry; musk, flower scents such as lavender-like, rose-like, iris-like, and carnation-like. Other pleasant scents include herbal and woodland scents derived from pine, spruce and other forest smells. Fragrances may also be derived from various oils, such as essential oils, or from plant materials such as peppermint, spearmint and the like.

A list of suitable fragrances is provided in U.S. Patent No. 4,534,891, the contents of which are incorporated by reference as if set forth in its entirety. Another source of suitable fragrances is found in Fragrances, Cosmetics and Soaps, Second Edition, edited by W. A. Poucher, 1959. Among the fragrances provided in this treatise are acacia, cassie, chypre, cyclamen, fern, gardenia, hawthorn, heliotrope, honeysuckle, hyacinth, jasmine, lilac, lily, magnolia, mimosa, narcissus, freshly-cut hay, orange blossom, orchid, reseda, sweet pea, trefle, tuberose, vanilla, violet, wallflower, and the like.

As used herein, olfactory effective amount is understood to mean the level of the fragrance-containing particles that will contribute its particular olfactory characteristics to a product. The olfactory effect of the composition will be the sum of the effects of each of the fragrance or fragrance ingredients including the fragrance-containing particles or fragrance-containing particles of the present invention. Thus, the particles of the invention can be used to alter the aroma characteristics of a product or fragrance composition by modifying the olfactory reaction contributed by another ingredient in the product or fragrance. Similarly, when flavors are employed the level of encapsulated flavor particles of the invention will vary depending on many factors including other ingredients, their relative amounts and the effect that is desired. Those with skill in the art will incorporate suitable materials in the invention when the product incorporating the present invention is intended for human or animal consumption.

Suitable conventional flavoring materials include saturated fatty acids, unsaturated fatty acids and amino acids; alcohols, including primary and secondary alcohols; esters; carbonyl compounds including ketones and aldehydes; lactones; other cyclic organic materials including benzene derivatives, acyclic compounds, heterocyclics such as furans, pyridines, pyrazines and the like; sulfur-containing compounds including thiols, sulfides, disulfides and the like; proteins; lipids, carbohydrates; so-called flavor potentiators such as monosodium glutamate, magnesium glutamate, calcium glutamate, guanylates and inosinates; natural flavoring materials such as cocoa, vanilla and caramel; essential oils and extracts such as anise oil, clove oil and the like and artificial flavoring materials such as vanillin, ethyl vanillin and the like.

Specific preferred flavor adjuvants include, but are not limited to, the following: anise oil; ethyl-2-methyl butyrate; vanillin; cis-3-heptenol; cis-3-hexenol; trans-2-heptenal; butyl valerate; 2,3-diethyl pyrazine; methyl cyclo-pentenolone; benzaldehyde; valerian oil; 3,4-dimeth-oxyphenol; amyl acetate; amyl cinnamate; y-butyryl lactone; furfural; trimethyl pyrazine; phenyl acetic acid; isovaleraldehyde; ethyl maltol; ethyl vanillin; ethyl valerate; ethyl butyrate; cocoa extract; coffee extract; peppermint oil; spearmint oil; clove oil; anethol; cardamom oil; wintergreen oil; cinnamic aldehyde; ethyl-2-methyl valerate; γ-hexenyl lactone; 2,4-decadienal; 2,4-heptadienal; methyl thiazole alcohol (4-methyl-5-β-hydroxyethyl thiazole); 2-methyl butanethiol; 4-mercapto-2-butanone; 3-mercapto-2-pentanone; 1-mercapto-2-propane; benzaldehyde; furfural; furfuryl alcohol; 2-mercapto propionic acid; alkyl pyrazine; methyl pyrazine; 2-ethyl-3-methyl pyrazine; tetramethyl pyrazine; polysulfides; dipropyl disulfide; methyl benzyl disulfide; alkyl thiophene; 2,3-dimethyl thiophene; 5-methyl furfural; acetyl furan; 2,4-decadienal; guiacol; phenyl acetaldehyde; β-decalactone; d-limonene; acetoin; amyl acetate; maltol; ethyl butyrate; levulinic acid; piperonal; ethyl acetate; n-octanal; n-pentanal; n-hexanal; diacetyl; monosodium glutamate; monopotassium glutamate; sulfur-containing amino acids, e.g., cysteine; hydrolyzed vegetable protein; 2-methylfuran-3-thiol;2-methyldihydrofuran-3-thiol; 2,5-dimethylfuran-3-thiol; hydrolyzed fish protein; tetramethyl pyrazine; propylpropenyl disulfide; propylpropenyl trisulfide; diallyl disulfide; diallyl trisulfide; dipropenyl disulfide; dipropenyl trisulfide; 4-methyl-2-[(methylthio)-ethyl]-1,3-dithiolane; 4,5-dimethyl-2-(methylthiomethyl)-1,3-dithiolane; and 4-methyl-2-(methylthiomethyl)-1,3-dithiolane. These and other flavor ingredients are provided in U.S. Patent Nos. 6,110,520 and 6,333,180.

The level of flavor or fragrance employed in the dry particle of the invention varies from about 5 to about 40 weight percent, preferably from about 15 to about 30 and most preferably from about 20 to about 25 weight percent.

In addition to the carrier, structuring material, and fragrance or flavor, other agents can be added to the particles by incorporation into the fragrance, structuring material, or the carrier. Well known materials such as solvents, surfactants and emulsifiers for the fragrance/flavor - structuring material mixture can be employed without departing from the scope of the present invention. Additionally, positively charged polymers can be added to either phase of the particle to promote deposition on hair, skin, cloth, or other negatively charged substrates. These materials include modified starches, quaternary salts, cationic guar gum, hydroxyl ethyl cellulose, and cationic silicones.

Optionally, functional materials such as hydrocolloids and other water-soluble polymers may be added to lend certain desired characteristics to the particle, such as creating a viscous micro-environment in aqueous solutions which can enhance particle integrity and reduce flavor/fragrance diffusion. Suitable hydrocolloids and other water-soluble polymers include gum arabic, alginate, carageenan and gelatin. The level of the functional carrier materials is from about 0.1 to about 20 weight percent on a dry basis, preferably from about 1 to about 10 weight percent and most preferably from about 2 to about 5 weight percent of the particle weight.

According to one embodiment of the invention, the carrier material is dissolved in a suitable solvent, commonly water. This action may form a true solution, as noted by clarity and lack of a precipitate, or may result in a form that may more properly be referred to as a sol, or a somewhat imperfect solution. The term "solution" used throughout this specification is meant to encompass each of these situations.

The most preferred drying method is spray drying, although other solvent removal techniques can be used.

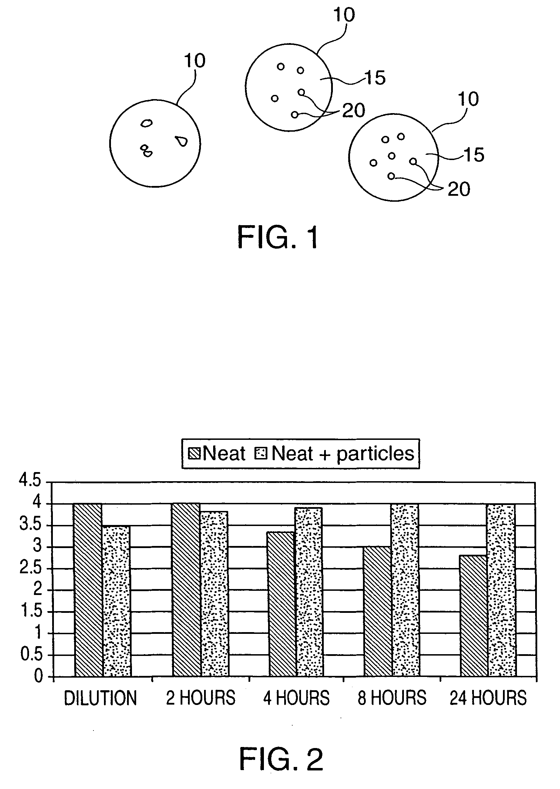

Referring to Figure 1, particle 10 is depicted as made in a spherical shape. A spherical shape is commonly achieved by a spray drying process. Figure 1 depicts the shell is comprised of a continuous water soluble material 15 such as a starch and the structuring material and fragrance such as ETPA - fragrance mixture or flavor mixture 20 as discrete phases located throughout the continuous water soluble material phase.

Particle size can vary depending on the conditions used to dry the particles, but the particle size generally ranges from about 10 to about 200 microns and more preferably from about 30 to about 100 microns. The size of the discrete flavor / fragrance particles within the particle are commonly from about 0.1 to about 25 microns, preferably from about 5 to about 20 microns. After the drying process is complete a grinding or sizing process can be employed if desired to achieve a more consistent and narrower particle size distribution.

The inventive particles are suitable for incorporation into many products. Some non-limiting examples include cosmetics, cleaning products such as bleaches, cleansers, detergents, shampoos, etc. As can be appreciated, the preferred embodiments will lend themselves to the use of powdered materials containing the particles of the invention. In a highly preferred embodiment, the powdered product is a laundry detergent.

The amount of the particles added to a product varies depending on the amount of fragrance that one might want to impart to a product. The level of particle can vary from about 0.5 up to about 20 weight percent of the product, preferably from about 1 to about 10 weight percent of the product.

The present invention is advantageously used in applications such as laundry detergents because the starch shell is soluble in water, and the structuring agent, such as ETPA is well suited for kinetically controlling fragrance release. For example, the ETPA aids in protecting the fragrance so that it can be delivered at a later or more appropriate time in the product usage. When a flavor is employed, the particles of the present invention can be added alone or with other spices and flavoring, with baking powders and baking sodas or in any other application in which a powder is added to a foodstuff. Suitable food products that that can employ the present invention include but are not limited to soups, salad dressings and yogurts.

Unless noted to the contrary, all percentages are given on a weight percent on a dry basis, and all temperatures are in Centigrade. The following examples are not meant to define or otherwise limit the scope of the invention. Rather the scope of the invention is to be ascertained according to the claims that follow the examples.

### EXAMPLE 1

A spray dried fragrance-containing material was prepared using the following materials:
300 grams deionized water;
120 grams CAPSUL starch [National Starch and Chemical Company];
40 grams of ETPA [Arizona Chemical]; and
40 grams of a fragrance formulation.

A preblend of starch and water was formed at 70°C. The fragrance oil and ETPA were mixed separately at 80°C and then added to the starch solution. The materials were blended for 2 minutes at 80°C before spray drying on an Armfield spray dryer while maintaining temperature of 80°C on the solution. 168 Grams of dry powder was obtained.

### EXAMPLE 2

A spray dried fragrance-containing material was prepared using the following materials:
280 grams deionized water;
112 grams M100 maltodextrin [available from Grain Processing Corporation];
28 grams gum Arabic [Colloides Naturels Inc.];
20 grams of ETPA [Arizona Chemical]; and
40 grams of a predominately aldehyde-containing fragrance formulation.

A preblend of M100, gum arabic and water was formed at 70°C. The fragrance oil and ETPA were mixed separately at 80°C and then added to the M100, gum arabic solution at 80°C until the ETPA was melted. The materials were homogenized for 5 minutes before spray drying on an Armfield spray dryer. 166 Grams of dry powder was obtained.

### EXAMPLE 3

A spray dried fragrance-containing material was prepared using the following materials:
320 grams deionized water; 120 grams CAPSUL starch [National Starch and Chemical Company];
40 grams of ETPA [Arizona Chemical]; and
40 grams of a fragrance formulation.

A preblend of starch and water was formed at 70°C. The fragrance oil and ETPA were mixed separately at 80°C until melted and then added to the starch solution. The materials were homogenized for 5 minutes at 80°C before spray drying on an Armfield spray dryer while maintaining a temperature of 80°C on the solution. 161 Grams of dry powder was obtained.

### EXAMPLE 4

A spray dried fragrance-containing material was prepared using the following materials:
300 grams deionized water;
120 grams CAPSUL starch [National Starch and Chemical Company];
40 grams of ETPA [Arizona Chemical];
20 grams of clay; and
40 grams of a fragrance formulation.

A preblend of starch, clay and water was formed at 70°C. The fragrance formulation and ETPA were mixed separately at 80°C and then added to the starch solution. The materials were homogenized for 10 minutes at 80°C before spray drying. 133 Grams of dry powder was obtained.

### EXAMPLE 5

A spray dried fragrance-containing material was prepared using the following materials:
300 grams deionized water;
100 grams CAPSUL starch
30 grams of ETPA [Arizona Chemical];
30 grams of Neobee [Stepan Company]; and
40 grams of a fragrance formulation.

A preblend of starch and water was formed at 70°C. The fragrance formulation, Neobee and ETPA were mixed separately at 80°C and then added to the starch solution. The materials were homogenized for 10 minutes at 80°C before spray drying. 172 Grams of dry powder was obtained.

### EXAMPLE 6

The particles of Example 1, containing ETPA, starch and fragrance, were added to a commercial fragrance-free powder detergent. Clothes were washed with a commercial detergent and the same commercial detergent with the composition of the present invention.

Panelists were asked to compare the fragrance imparted to the clothes soaking in a wash basin containing water plus the commercial product alone as compared to the commercial product and the present invention together. The products were provided such that the control, the commercial detergent, contained the same level of fragrance as the commercial detergent and the fragrance-containing particles of the present invention. Clothes were washed under identical conditions using the two detergent systems. Figure 2 displays the results of this evaluation. Neat indicates that the detergent did not include the particles of the present invention. Neat + particles indicates that the detergent also contained the particles of the present invention. Results indicate that with increasing soak time the particles imparted a fragrance with a longer-lasting and more powerful bloom.

Panelists were then asked to compare the fragrance imparted to the washed, undried clothes. The damp clothes were evaluated by 20 panelists. More than 80 percent of the panelists chose the clothes laundered in the detergent and the particles of the present invention compared to a control detergent containing the same level of fragrance.

As noted, the above description is a non-limiting example of certain preferred particles of the invention and methods for making them. Other obvious variants will be apparent to those skilled in the art without parting from the scope and spirit of the invention as claimed herein.

## Claims

1. A particle composition comprising:
a water soluble carrier,
a structuring agent, and
flavor or fragrance.

2. The particle composition of claim 1 having a particle size of from 30 to 80 microns.

3. The particle composition of claim 1 or claim 2, which additionally comprises a clay material.

4. The particle composition of any one of claims 1 to 3, wherein the water soluble carrier is starch and the level of starch is from 25 to 75% of the particle.

5. The particle composition of any one of claims 1 to 4, wherein the structuring agent is an ester terminated polyamide and the level of ester terminated polyamide is from 5 to 40 weight percent of the particle.

6. The particle composition of any one of claims 1 to 5, containing a fragrance material, wherein the fragrance level is from 5 to 30 weight percent of the particle.

7. The particle composition of any one of claims 1 to 6, wherein the structuring agent and flavor or fragrance materials are found in discrete beads in the water-soluble carrier.

8. The particle of claim 7 wherein the discrete beads have a particle size of from 0.1 to 25 microns.

9. The particle of claim 8 wherein the bead comprises an ester terminated polyamide and fragrance material.

10. The particle of claim 8 wherein the bead comprises a fat or wax structuring agent and a fragrance material.

11. A method for making the particle composition of claim 1 wherein:
the water soluble carrier is added to a suitable solvent to form a first admixture;
a structuring agent is added to a fragrance or flavor to form a second admixture; the first and second admixtures are combined while being subjected to high speed mixing or homogenization; and
the final admixture is dried to form a particle.

12. A powdered product containing the particle composition of any one of claims 1 to 10.

13. The powdered product of claim 12 which is a cosmetic, detergent, bleach, deodorizer or shampoo.
